# EUROPEAN PATENT APPLICATION

(11) **EP 0 785 195 A1**
(43) Date of publication of application: **23.07.1997**
(21) Application number: 97300201.7
(22) Date of filing: 14.01.1997
(51) Int. Cl.: C07D 401/14, A61K 31/44

(54) **Piperidine or tetrahydropyridine derivatives having affinity for the serotonin 5-HTIA receptor**

(30) Priority: 16.01.1996 US 10050
(71) Applicant: AMERICAN HOME FOOD PRODUCTS, INC., Madison, New Jersey 07940-0874 (US)
(72) Inventor: Baudy, Reinhardt Bernhard, Doylestown, Pennsylvania, 18901 (US)
(74) Representative: Connelly, Michael John

(57) **Abstract**

This invention provides compounds having the structure 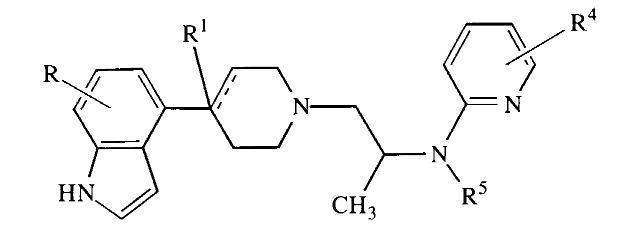 wherein
R and R⁴ are each, independently, hydrogen, -CN, -OR², -COR², -COOR², -CONR²R³, alkyl, alkenyl, alkynyl, perhaloalkyl, or halogen;
the dashed line indicates an optional double bond;
R¹ is hydrogen, -OH, -OR², or is absent when the double bond is present;
R² and R³ are each, independently, alkyl, alkenyl, alkynyl, phenyl, or phenylalkyl;
R⁵ is hydrogen, alkyl of 1-6 carbon atoms, or -COR⁶.
R⁶ is alkyl, alkenyl, alkynyl, cycloalkyl, phenylalkyl, or Ar; and
Ar is an aryl or heteroaryl radical which may be optionally substituted or a pharmaceutically acceptable salt thereof. The compounds are useful as antipsychotic, antidepressant and anxiolytic agents useful in the treatment and relief of the symptoms of these disease states.

## Description

This invention relates to piperidine or tetrahydropgridine derivatives and pharmaceutical compositions containing them. The compounds are useful as pharmaceuticals. In particular, they have selectivity for the serotonergic 5-HT_{1A} receptor and are useful in the treatment of central nervous system disorders. The compounds are selected from those having the formula 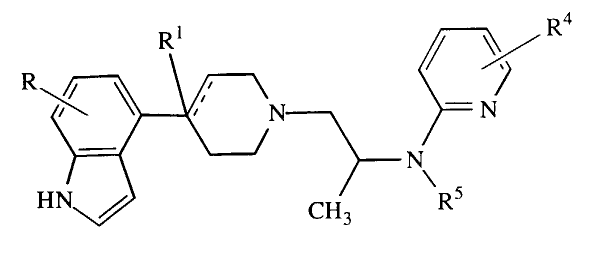 wherein
- R and R⁴: are each, independently, hydrogen, -CN, -OR², -COR², -COOR², -CONR²R³, alkyl of 1-6 carbon atoms, alkenyl of 2-7 carbon atoms, alkynyl of 2-7 carbon atoms, or halogen;
the dashed line indicates an optional double bond;
- R¹: is hydrogen, -OH, -OR², or is absent when the double bond is present;
- R² and R³: are each, independently, alkyl of 1-7 carbon atoms, alkenyl of 2-7 carbon atoms, alkynyl of 2-7 carbon atoms, phenyl, or phenylalkyl of 7-10 carbon atoms;
- R⁵: is hydrogen, alkyl of 1-6 carbon atoms, or -COR⁶;
- R⁶: is alkyl of 1-6 carbon atoms, alkenyl of 2-7 carbon atoms, alkynyl of 2-7 carbon atoms, cycloalkyl of 3-10 carbon atoms, phenylalkyl of 7-10 carbon atoms, or Ar; and
- Ar: is an aryl or heteroaryl radical which may be optionally mono-, di-, or tri-substituted with a group selected from alkyl of 1-6 carbon atoms, alkenyl of 2-7 carbon atoms, alkynyl of 2-7 carbon atoms, alkoxy of 1-6 carbon atoms, cyano, halo, hydroxy, nitro, carbalkoxy of 2-7 carbon atoms, trifluoromethyl, trifluoromethoxy, amino, dialkylamino of 1-6 carbon atoms per alkyl group, dialkylaminoalkyl of 3-12 carbon atoms, hydroxyalkyl of 1-6 carbon atoms, alkoxyalkyl of 2-12 carbon atoms, alkylthio of 1-6 carbon atoms, -SO₃H, and -CO₂H;
and their pharmaceutically acceptable salts.

The pharmaceutically acceptable salts include acid addition salts. The acid addition salts may be derived from such organic and inorganic acids as: acetic, lactic, citric, tartaric, succinic, maleic, malonic, gluconic, hydrochloric, hydrobromic, phosphoric, nitric, sulfuric, methanesulfonic, and similarly known acceptable acids.

The terms alkyl of 1-6 carbon atoms, alkenyl of 2-7 carbon atoms, and alkynyl of 2-7 carbon atoms, include both straight chain as well as branched carbon chains. The term "halogen" refers to fluoro, chloro, bromo, or iodo. It is preferred that the cycloalkyl ring be of 4-7 carbon atoms.

It is preferred that the aryl and heteroaryl radicals of Ar are phenyl, pyridyl, furyl, pyrrolyl, thiophenyl, imidazolyl, oxazolyl, or thiazolyl that may be optionally mono-, di-, or tri-substituted with a group selected from alkyl of 1-6 carbon atoms, alkenyl of 2-7 carbon atoms, alkynyl of 2-7 carbon atoms, alkoxy of 1-6 carbon atoms, cyano, halo, hydroxy, nitro, carbalkoxy of 2-7 carbon atoms, trifluoromethyl, trifluoromethoxy, amino, dialkylamino of 1-6 carbon atoms per alkyl group, dialkylaminoalkyl of 3-12 carbon atoms, hydroxyalkyl of 1-6 carbon atoms, alkoxyalkyl of 2-12 carbon atoms, alkylthio of 1-6 carbon atoms, -SO₃H, and -CO₂H.

The compounds within the scope of the invention by virtue of their configuration, have one or more asymmetric centers and therefore exhibit stereoisomerism. Such centers can contain either the R or S configuration or can be racemic with respect to such center or centers.

Of these compounds, the preferred members are those in which R⁵ is hydrogen; those in which R⁵ is COR⁶; and those in which R⁵ is COR⁶ and R⁶ is cycloalkyl of 7-10 carbon atoms.

The compounds of this invention can be prepared by conventional methods from starting materials that are either commercially available or can be made by methods disclosed in the literature. For example, as shown below, an appropriately N-protected 4-bromo-indole is lithiated and reacted with 4-piperidone benzylcarbamate. Deprotection can be accomplished using selective hydrogenation to afford the desired 4-hydroxy-4-indol-4-yl-piperidine (1). 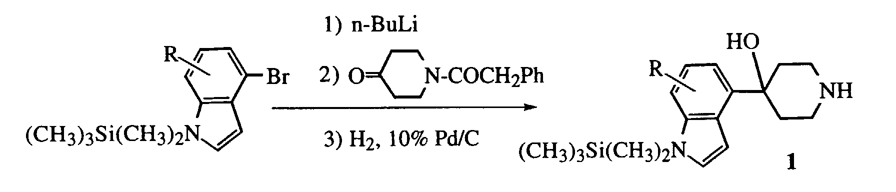 The 4-substituted piperidine can then be condensed with an appropriately substituted cyclic sulfamate to yield compound 2. The cyclic sulfamate and its preparation are disclosed in WO 95/33725. 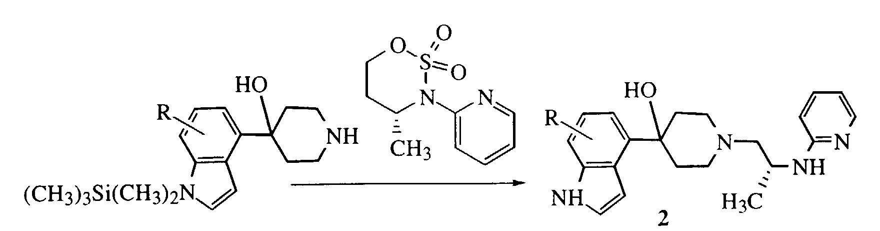 The desired compound (3) can be obtained by dehydration and subsequent acylation with an appropriately substituted acid chloride.

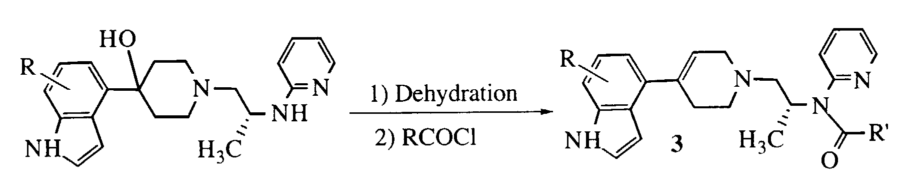

Other compounds of this invention can be prepared using the methodology described above using appropriately substituted starting materials and reactants.

Representative compounds of this invention were evaluated and determined to have high affinity for the serotonin 5-HT_{1A} receptor by evaluating the compound's ability to displace [³H] 8-OHDPAT (dipropylaminotetralin) from the 5-HT_{1A} serotonin receptor following the procedure of Hall et al., J. Neurochem. 44, 1685 (1985). This standard pharmacological test procedure was employed to analogize this property of the claimed compounds with that of buspirone, which is a standard for anxiolytic activity, and, like the compounds of this invention, displays potent affinity for the 5-HT_{1A} serotonin receptor subtype. The anxiolytic activity of buspirone is believed to be, at least partially, due to its 5-HT_{1A} receptor affinity (Vander Maclen et al., Eur. J. Pharmacol. 1986, 129 (1-2) 133-130). In this standard pharmacological test procedure, buspirone has an IC₅₀ of approximately 10 nM.

The results obtained for representative compounds of this invention in the standard pharmacological test procedure described above, are as follows:

| Compound | 5-HT_{1A}Binding (IC₅₀) |
|---|---|
| Example 1 | 11.6 nM |
| Example 2 | 1.7 nM |

The results obtained in the standard pharmacological test procedure demonstrate that the compounds this invention possess high affinities for the serotonin 5-HT_{1A} receptor, and consequently, they are useful in the treatment of multi-CNS disorders amenable to treatment with antipsychotic, antidepressant and anxiolytic agents. As such, the compounds of this invention may be administered a mammal in need of antipsychotic, antidepressant and/or anxiolytic medical treatment in an amount sufficient to alleviate the symptoms of the disease state, such as depression, paranoia, schizophrenia, anxiety, sleep disorders, eating disorders, cognitive disorders, panic, social phobia, obsessive compulsive disorders, sexual dysfunction, addiction, and related problems. When administered for the treatment of the above disease states, the compounds of this invention can be administered to a mammal orally, parenterally, intranasally, intrabronchially, transdermally, intravaginally, or rectally.

The invention includes a compound of this invention for use as a pharmaceutical and also pharmaceutical composition containing such a compound in association or combination with a pharmaceutical carrier. The compound can be formulated neat or with a pharmaceutical carrier to a mammal in need thereof. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellant.

Liquid pharmaceutical compositions which are sterile solutions or suspensions can be utilized by, for example, intramuscular, intraperitoneal or subcutaneous injection. Sterile solutions can also be administered intravenously. The compound can also be administered orally either in liquid or solid composition form.

The compounds of this invention may be administered rectally in the form of a conventional suppository. For administration by intranasal or intrabronchial inhalation or insufflation, the compounds of this invention may be formulated into an aqueous or partially aqueous solution, which can then be utilized in the form of an aerosol. The compounds of this invention may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipermiable membrane covering a reservoir containing the active ingredient with or without a carrier, or a matrix containing the active ingredient. Other occlusive devices are known in the literature.

The dosage to be used in the treatment of a specific psychosis must be subjectively determined by the attending physician. The variables involved include the specific psychosis or state of anxiety or depression and the size, age and response pattern of the patient. Based on the results obtained in the standard pharmacological test procedures, projected oral daily dosages of active compound would be 1-100 mg/kg, preferably between 1-30 mg/kg, and more preferably between 1-10 mg/kg. Projected intravenous daily dosages would be 0.2-20 mg/kg, preferably between 0.2-6 mg/kg, and more preferably between 0.2-2 mg/kg. Treatment will generally be initiated with small dosages less than the optimum dose of the compound. Thereafter the dosage is increased until the optimum effect under the circumstances is reached; precise dosages for oral, parenteral, nasal, or intrabronchial administration will be determined by the administering physician based on experience with the individual subject treated. Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form.

The following examples illustrate the production of representative compounds of this invention.

### Example 1

### {(R)-2-[4-(1H-Indol-4-yl)-(1,2,3,6-tetrahydro-pyridin-1-yl)]-1-methyl-ethyl}-(pyridin-2-yl)-amine

A solution of commercially available 4-bromoindole (10 mmole, 2.084 g) in dry N,N-dimethylformamide (DMF, 5 mL) was added dropwise to a stirred suspension of sodium hydride (60%, 12 mmole, 0.48 g) in dry DMF (15 mL) at - 10°C. The reaction mixture was stirred for 30 minutes at -10°C after which t-butyldimethylsilyl chloride (11 mmole, 1.658 g) was added in a portionwise fashion. The reaction mixture was allowed to reach ambient temperature, at which point it was poured into ice cold water (70 mL) and the crude product extracted with methylene chloride (3 x 60 mL). The combined organic layer was dried over magnesium sulfate, filtered, and evaporated to dryness in vacuo. Column flash chromatography of the residue on 200 g of silica gel with chloroform / hexane as eluant yielded 2.2 g of the N-protected 4-bromoindole which was dissolved in dry tetrahydrofuran (THF, 100 mL).

At -78°C under an atmosphere of dry nitrogen, n-butyllithium (2.5 M, 13.4 mmole, 5.4 mL) was added to the solution via a syringe and stirring was continued for 30 minutes at -78°C. A solution of 1-(N-benzyloxycarbonyl)-4-piperidone (6.8 mmole, 1.586 g) in dry THF (40 mL) was added at -30°C and stirring continued at ambient temperature for 12 hours. The reaction mixture was then poured into ice cold water (150 mL) and the crude product extracted with ether (3 x 100 mL). The combined organic layer was dried over magnesium sulfate, filtered and evaporated to dryness. The residue was flash chromatographed on 160 g silica gel using 2% methanol / chloroform as eluant affording 1.15 g of the desired piperidine intermediate which was then hydrogenated for three hours at atmospheric pressure in ethanol (80 mL) in the presence of 10% palladium on carbon (120 mg). The reaction mixture was purged with nitrogen, the catalyst filtered off and the filtrate evaporated in vacuo to yield the deprotected hydroxypiperidine derivative as a colorless oil.

A solution of the hydroxypiperidine (2.87 mmole, 950 mg) in acetonitrile (20 mL) was treated with (R)-4-methyl-3-pyridin-2-yl-[1,2,3]oxathiazinane-2,2-dioxide (3 mmole, 631 mg) at ambient temperature for 2 hours. The resultant mixture contained {(R)-2-[4-(1H-indol-4-yl)-4-hydroxypiperidin-1-yl]-1-methyl-ethyl}-(pyridin-2-yl)amine. The mixturewas evaporated in vacuo and the residue dissolved in THF (10 mL) and water (10 mL). Concentrated sulfuric acid (0.15 mL) was added dropwise over 1 minute after which the mixture was stirred another 30 minutes at ambient temperature. Thereafter the pH was adjusted to 7 with the addition of sodium hydrogencarbonate powder. Stirring was continued for another hour. The reaction mixture was then diluted with ethyl acetate and water. The organic layer was separated, washed with brine, dried over magnesium sulfate, filtered and the filtrate evaporated to dryness.

The residue was refluxed in acetic acid (60 mL) for 3 hours, evaporated in vacuo and the residue partitioned between aqueous sodium hydrogencarbonate (50 mL) and chloroform (80 mL). The organic layer was separated, dried over magnesium sulfate and evaporated. The residue was subjected to flash column chromatography on silica gel (50 g). Elution with 3% methanol / chloroform gave 300 mg of an oil, some of which was converted in chloroform with the addition of ethereal HCl to the title compound, obtained as red brown microcrystals, mp 102-4°C.

| Elemental Analysis for: C₂₁H₂₄N₄·2.8HCl·1H₂O. | | | |
|---|---|---|---|
| Calcd: | C, 55.73; | H, 6.41; | N, 12.38. |
| Found: | C, 56.10; | H, 6.73; | N, 11.92. |

### EXAMPLE 2

### (R)-N-({2-[4-(1H-indol-4-yl)1,2,3,6-tetrahydro-2H-pyridin-1-yl]-1-methyl-ethyl}-N-(pyridin-2-yl)-cyclohexanecarboxamide

A solution of cyclohexanecarbonyl chloride (0.6 mmole, 88 mg) in methylene chloride (10 mL) was added dropwise at 0°C to a solution of the compound of Example 1 (0.54 mmole, 180 mg) in methylene chloride (10 mL). The reaction mixture was allowed to reach ambient temperature and stirring was continued for 16 hours. The obtained solution was washed with 2N sodium hydroxide (15 mL), the organic layer separated, dried over magnesium sulfate, filtered and the filtrate evaporated to dryness. The obtained residue was subjected to flash column chromatography on silica gel (10g). Elution with 5% methanol in chloroform gave an off-white foam which was dissolved in ethanol (3 mL) and treated at once with a solution of maleic acid (0.338 mmole, 39 mg) in ethanol (2 mL). The obtained solution was concentrated in vacuo and triturated with ether affording 120 mg of the title compound as light amber microcrystals, mp 70-3°C.

| Elemental Analysis for: C₂₈H₃₄N₄O·1.7C₄H₄O₄·0.1CHCl₃. | | | |
|---|---|---|---|
| Calcd: | C, 64.12; | H, 6.31; | N, 8.59. |
| Found: | C, 64.50; | H, 6.64; | N, 8.00. |

## Claims

1. A compound having the structure 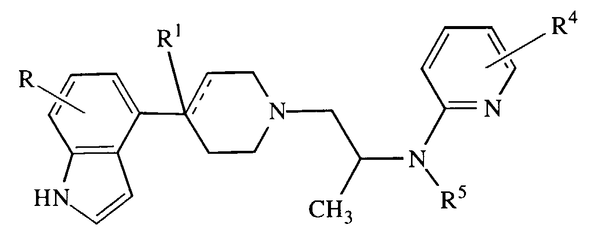 wherein
R and R⁴ are each, independently, hydrogen, -CN, -OR², -COR², -COOR², -CONR²R³, alkyl of 1-6 carbon atoms, alkenyl of 2-7 carbon atoms, alkynyl of 2-7 carbon atoms, perhaloalkyl of 1-6 carbon atoms, or halogen;
the dashed line indicates an optional double bond;
R¹ is hydrogen, -OH, -OR², or is absent when the double bond is present;
R² and R³ are each, independently, alkyl of 1-7 carbon atoms, alkenyl of 2-7 carbon atoms, alkynyl of 2-7 carbon atoms, phenyl, or phenylalkyl of 7-10 carbon atoms;
R⁵ is hydrogen, alkyl of 1-6 carbon atoms, or -COR⁶;
R⁶ is alkyl of 1-6 carbon atoms, alkenyl of 2-7 carbon atoms, alkynyl of 2-7 carbon atoms, cycloalkyl of 3-10 carbon atoms, phenylalkyl of 7-10 carbon atoms, or Ar; and
Ar is an aryl or heteroaryl radical which may be optionally mono-, di-, or tri-substituted with a group selected from alkyl of 1-6 carbon atoms, alkenyl of 2-7 carbon atoms, alkynyl of 2-7 carbon atoms, alkoxy of 1-6 carbon atoms, cyano, halo, hydroxy, nitro, carbalkoxy of 2-7 carbon atoms, trifluoromethyl, trifluoromethoxy, amino, dialkylamino of 1-6 carbon atoms per alkyl group, dialkylaminoalkyl of 3-12 carbon atoms, hydroxyalkyl of 1-6 carbon atoms, alkoxyalkyl of 2-12 carbon atoms, alkylthio of 1-6 carbon atoms, -SO₃H, and -CO₂H;
or a pharmaceutically acceptable salt thereof.

2. The compound of claim 1, wherein R⁵ is hydrogen or a pharmaceutically acceptable salt thereof.

3. The compound of claim 1, wherein R⁵ is -COR⁶ or a pharmaceutically acceptable salt thereof.

4. The compound of claim 2, wherein R⁶ is cycloalkyl of 3-10 carbon atoms or a pharmaceutically acceptable salt thereof.

5. (R)-2-[4-(1H-Indol-4-yl)-(1,2,3,6-tetrahydro-pyridin-1-yl)]-1-methyl-ethyl}(pyridin-2-yl)-amine or a pharmaceutically acceptable salt thereof.

6. (R)-2-[4-(1H-Indol-4-yl)-(4-hydroxypiperidin-1-yl)-amine or a pharmaceutically acceptable salt thereof.

7. (R)-N-({2-[4-(1H-Indol-4-yl)1,2,3,6-tetrahydro-2H-pyridin-1-yl]-1-methyl-ethyl}-N-(pyridin-2-yl)-cyclohexanecarboxamide or a pharmaceutically acceptable salt thereof.

8. A pharmaceutical composition which comprises a compound of the structure 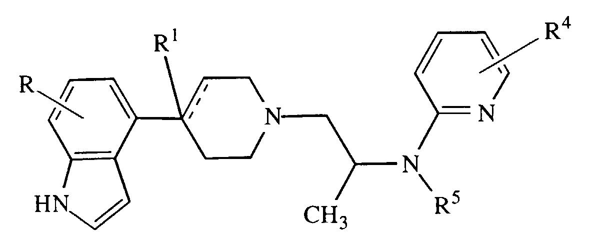 wherein
R and R⁴ are each, independently, hydrogen, -CN, -OR², -COR², -COOR², -CONR²R³, alkyl of 1-6 carbon atoms, alkenyl of 2-7 carbon atoms, alkynyl of 2-7 carbon atoms, perhaloalkyl of 1-6 carbon atoms, or halogen;
the dashed line indicates an optional double bond;
R¹ is hydrogen, -OH, -OR², or is absent when the double bond is present;
R² and R³ are each, independently, alkyl of 1-7 carbon atoms, alkenyl of 2-7 carbon atoms, alkynyl of 2-7 carbon atoms, phenyl, or phenylalkyl of 7-10 carbon atoms;
R⁵ is hydrogen, alkyl of 1-6 carbon atoms, or -COR⁶;
R⁶ is alkyl of 1-6 carbon atoms, alkenyl of 2-7 carbon atoms, alkynyl of 2-7 carbon atoms, cycloalkyl of 3-10 carbon atoms, phenylalkyl of 7-10 carbon atoms, or Ar; and
Ar is an aryl or heteroaryl radical which may be optionally mono-, di-, or tri-substituted with a group selected from alkyl of 1-6 carbon atoms, alkenyl of 2-7 carbon atoms, alkynyl of 2-7 carbon atoms, alkoxy of 1-6 carbon atoms, cyano, halo, hydroxy, nitro, carbalkoxy of 2-7 carbon atoms, trifluoromethyl, trifluoromethoxy, amino, dialkylamino of 1-6 carbon atoms per alkyl group, dialkylaminoalkyl of 3-12 carbon atoms, hydroxyalkyl of 1-6 carbon atoms, alkoxyalkyl of 2-12 carbon atoms, alkylthio of 1-6 carbon atoms, -SO₃H, and -CO₂H;
or a pharmaceutically acceptable salt thereof, in association or combination with a pharmaceutical carrier.

9. A pharmaceutical composition as claimed in claim 8, containing a compound as claimed in claim 2 or 3.

10. A pharmaceutical composition as claimed in claim 8, containing a compound as claimed in any one of claims 4 to 6.

11. A compound as claimed in any one of claims 1 to 7, for use as a pharmaceutical.
